# EUROPEAN PATENT SPECIFICATION

(11) **EP 3 880 207 B1**
(45) Date of publication and mention of the grant of the patent: **27.03.2024**
(21) Application number: 19809391.6
(22) Date of filing: 14.11.2019
(51) Int. Cl.: A61K 31/519, A61K 31/553, A61K 31/704, A61K 31/706, A61P 35/00, A61P 35/02

(54) **COMBINATION OF A MCL-1 INHIBITOR AND MIDOSTAURIN, USES AND PHARMACEUTICAL COMPOSITIONS THEREOF**
KOMBINATION AUS EINEM MCL-1-INHIBITOR UND MIDOSTAURIN, VERWENDUNGEN DAVON UND PHARMAZEUTISCHE ZUSAMMENSETZUNGEN DARAUS
COMBINAISON D'UN INHIBITEUR DE MCL-1 ET DE MIDOSTAURINE, UTILISATIONS ET COMPOSITIONS PHARMACEUTIQUES ASSOCIÉES

(30) Priority: 14.11.2018 US 201862767007 P; 27.03.2019 US 201962824515 P; 08.10.2019 US 201962912160 P
(43) Date of publication of application: 22.09.2021
(73) Proprietor: Les Laboratoires Servier, 92284 Suresnes Cedex (FR); Novartis AG, 4056 Basel (CH)
(72) Inventor: HALILOVIC, Ensar, Cambridge, MA 02139 (US); WANG, Youzhen, Cambridge, MA 02139 (US); MORRIS, Erick, Cambridge, MA 02139 (US); KONOPLEVA, Marina, Houston, TX 77030 (US); SKWARSKA, Anna, Houston, TX 77030 (US)
(74) Representative: Bernad, Stéphane
(86) International application number: PCT/EP2019/081291
(87) International publication number: WO 2020/099542

(56) References cited:
- WO-A1-2015/097123
- WO-A1-2018/078064
- WO-A1-2018/126898
- ANDRÁS KOTSCHY ET AL: "The MCL1 inhibitor S63845 is tolerable and effective in diverse cancer models", NATURE, vol. 538, no. 7626, 19 October 2016 (2016-10-19), pages 477-482, XP055571783, London ISSN: 0028-0836, DOI: 10.1038/nature19830
- MARLISE R. LUSKIN ET AL: "Midostaurin/PKC412 for the treatment of newly diagnosed FLT3 mutation-positive acute myeloid leukemia", EXPERT REVIEW OF HEMATOLOGY, vol. 10, no. 12, 2 December 2017 (2017-12-02), pages 1033-1045, XP055665097, UK ISSN: 1747-4086, DOI: 10.1080/17474086.2017.1397510

## Description

### FIELD OF THE INVENTION

The present invention relates to a combination of Midostaurin, a multi-targeted tyrosine kinase inhibitor as defined below, and a Mcl-1 inhibitor which is Compound B or Compound C. as defined below.

The invention also relates to said combination for use in the treatment of cancer, in particular acute myeloid leukaemia, and a pharmaceutical formulation suitable for the administration of such combination.

### BACKGROUND

Midostaurin is also named as 4'-N-benzoylstaurosporine or PKC412. The chemical structure of Midostaurin is shown below:

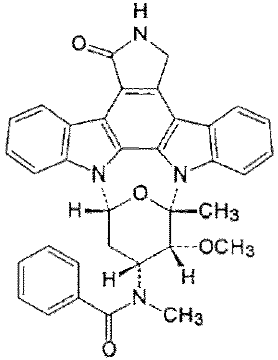

Midostaurin, which is also referred to herein as Compound A, its synthesis, its use in the treatment of cancer and pharmaceutical formulations thereof, are described in U.S. Patent No. 5,093,330. Midostaurin is specifically described in Example 18 of U.S. Patent No. 5,093,330. As a single agent against solid tumors in a Phase I trial Midostaurin showed low toxicity but limited efficacy (Propper et al., Journal of Clinical Oncology 2001, 19, 1485-1492). When added to a standard of care treatment of cytarabine and daunorubicin induction and cytarabine consolidation, Midostaurin showed statically significant efficacy and increased overall survival in patients with AML with FLT3 mutation (Stone et al., New England Journal of Medicine 2017, 377, 454-464; Luskin et al., Expert Review of Hematology 2017, 10, 1033-1045).

Midostaurin was approved by the US FDA in April 2017 as the active ingredient of RYDAPT^{™} for the treatment of newly diagnosed acute myeloid leukaemia (AML) patients harboring a *FLT3* mutation, in combination with cytarabine and daunorubicin induction and cytarabine consolidation.

Cytarabine and daunorubicin induction followed by cytarabine consolidation is a standard of care for AML. Cytarabine and daunorubicin are chemotherapeutic drugs that act by killing proliferating cells. Cytarabine is an inhibitor of DNA polymerase and daunorubicin an anthracycline that blocks replication. Administration of cytarabine or daunorubicin can cause nausea and vomiting, as well as more severe side effects. Both drugs must be given by injection and should be given at specialized facilities so that patients can be closely monitored after administration.

The Mcl-1 inhibitor of the present invention is Compound B: (2*R*)-2-[(5*Sₐ*)-5-{3-chloro-2-methyl-4-[2-(4-methylpiperazin-1-yl)ethoxy]phenyl}-6-(4-fluorophenyl)thieno[2,3-*d*]pyrimidin-4-yl]oxy}-3-(2-{[2-(2-methoxyphenyl)pyrimidin-4-yl]methoxy}phenyl)propanoic acid, or Compound C: (2*R*)-2-{[(5*Sₐ*)-5-{3-chloro-2-methyl-4-[2-(4-methylpiperazin-1-yl)ethoxy]phenyl}-6-(5-fluorofuran-2-yl)thieno[2,3-*d*]pyrimidin-4-yl]oxy}-3-(2-{[1-(2,2,2-trifluoroethyl)-1*H*-pyrazol-5-yl]methoxy}phenyl)propanoic acid as described herein. Compound B and Compound C, their synthesis, their use in the treatment of cancer and pharmaceutical formulations thereof, are described in WO 2015/097123, WO 2016/207216, WO 2016/207217, WO 2016/207225, WO 2016/207226, WO 2017/125224, WO 2018/126898 and WO 2018/078064.

In particular, the Mcl-1 inhibitor is Compound B:
(2*R*)-2-{[(5*Sₐ*)-5-{3-chloro-2-methyl-4-[2-(4-methylpiperazin-1-yl)ethoxy]phenyl}-6-(4-fluorophenyl)thieno[2,3-*d*]pyrimidin-4-yl]oxy}-3-(2-{[2-(2-methoxyphenyl)pyrimidin-4-yl]methoxy}phenyl)propanoic acid, or a pharmaceutically acceptable salt thereof, which is described in Example 30 of WO 2015/097123.

In another embodiment, the Mcl-1 inhibitor is Compound C (S63845):
(2*R*)-2-{[(5*Sₐ*)-5-3-chloro-2-methyl-4-[2-(4-methylpiperazin-1-yl)ethoxy]phenyl}-6-(5-fluorofuran-2-yl)thieno[2,3-*d*]pyrimidin-4-yl]oxy}-3-(2-{[1-(2,2,2-trifluoroethyl)-1*H-*pyrazol-5-yl]methoxy}phenyl)propanoic acid, or a pharmaceutically acceptable salt thereof, which is described in Example 185 of WO 2015/097123.

Apoptosis is a highly regulated cell death pathway that is initiated by various cytotoxic stimuli, including oncogenic stress and chemotherapeutic agents. It has been shown that evasion of apoptosis is a hallmark of cancer and that efficacy of many chemotherapeutic agents is dependent upon the activation of the intrinsic mitochondrial pathway. Three distinct subgroups of the Bcl-2 family proteins control the intrinsic apoptosis pathway: (i) the pro-apoptotic BH3 (the Bcl-2 homology 3)-only proteins; (ii) the pro-survival members such as Bcl-2 itself, Bcl-xl, Bcl-w, Mcl-1 and Bcl-2a1; and (iii) the pro-apoptotic effector proteins BAX and BAK (Czabotar et al., Nature Reviews Molecular Cell Biology 2014, 15, 49-63). Overexpression of the anti-apoptotic members of Bcl-2 family is observed in many cancers, particularly in hematological malignancies such as mantle cell lymphoma (MCL), follicular lymphoma/diffuse large B-cell lymphoma (FL/DLBCL) and multiple myeloma (Adams and Cory, Oncogene 2007, 26, 1324-1337). Pharmacological inhibition of the anti-apoptotic proteins Bcl-2, Bcl-xl, Bcl-w and Mcl-1 by the recently developed BH3-mimetics drugs such as ABT-199 (venetoclax), ABT-263 (navitoclax) and S63845 has emerged as a therapeutic strategy to induce apoptosis and cause tumor regression in cancer (Zhang et al., Drug Resist. Updat. 2007, 10, 207-217; Kotschy et al., Nature 2016, 538, 477-482). Nevertheless, mechanisms of resistance to BH3 mimetics have been observed (Choudhary et al., Cell Death and Disease 2015, 6, e1593) and the use of combination therapies could improve efficacy and delay or even abrogate resistance development.

Acute myeloid leukaemia (AML) is a rapidly fatal blood cancer arising from clonal transformation of hematopoietic stem cells resulting in paralysis of normal bone marrow function and deaths due to complications from profound pancytopenia. AML accounts for 25 % of all adult leukaemias, with the highest incidence rates occurring in the United States, Australia and Europe (WHO. GLOBOCAN 2012. Estimated cancer incidence, mortality and prevalence worldwide in 2012. International Agency for Research on Cancer). Globally, there are approximately 88,000 new cases diagnosed annually. AML continues to have the lowest survival rate of all leukaemias, with expected 5-year survival of only 24 %. Although the standard therapy for AML (cytarabine in combination with an anthracycline, such daunorubicin) was conceived over 4 decades ago, the introduction of successful targeted therapies for this disease has remained an elusive goal. The concept of targeted therapy in AML has been hampered by the realisation that this disease evolves as a multi-clonal hierarchy, with rapid outgrowth of leukaemic sub-clones as a major cause of drug resistance and disease relapse (Ding et al., Nature 2012, 481, 506-510).

Activating mutations of *FLT3* (mostly *FLT3-ITD*) are present in about 30 % of AML patients at diagnosis, which lead to the subsequent activation of downstream signaling cascade including FLT3/JAK/STAT, MAPK and PI3K signaling pathways. The activation of FLT3 signaling suppresses apoptosis signals through inducing the phosphorylation of pro-apoptotic protein BAD and the expression of anti-apoptotic Bcl2 family members. Inhibition of FLT3-ITD kinase activity induces apoptosis via down-regulation of BAD phosphorylation and the expression of Bcl-xL in FLT3-ITD cells (Minami et al., Blood 2003, 102, 2969-2975).

There remains a need for new treatments and therapies for the treatment of cancer. It has unexpectedly been found that Midostaurin, in combination with Compound B or Compound C as described herein, interact in synergistic manner to strongly inhibit cell proliferation, in particular for AML, and more particularly for *FLT3 mutant* AML.

### SUMMARY OF THE INVENTION

According to a first aspect of the invention, there is provided a novel combination of
(a) Midostaurin, or a pharmaceutically acceptable salt thereof, or a complex thereof, or a co-crystal thereof, or a solvate, including hydrate, thereof, and
(b) a Mcl-1 inhibitor which is Compound B: (2*R*)-2-{[(5*Sₐ*)-5-{3-chloro-2-methyl-4-[2-(4-methylpiperazin-1-yl)ethoxy]phenyl}-6-(4-fluorophenyl)thieno[2,3-d]pyrimidin-4-yl]oxy}-3-(2-{[2-(2-methoxyphenyl)pyrimidin-4-yl]methoxy}phenyl)propanoic acid, or Compound C: (2*R*)-2-{[(5*Sₐ*)-5-{3-chloro-2-methyl-4-[2-(4-methylpiperazin-1-yl)ethoxy]phenyl}-6-(5-fluorofuran-2-yl)thieno[2,3-*d*]pyrimidin-4-yl]oxy}-3-(2-{[1-(2,2,2-trifluoroethyl)-1*H*-pyrazol-5-yl]methoxy}phenyl)propanoic acid,

or addition salts thereof with a pharmaceutically acceptable acid or base,
suitable for simultaneous, sequential or separate use.

In another aspect, the invention provides a combination as described herein, for use in the treatment of cancer, in particular acute myeloid leukaemia.

In a further aspect, the invention provides a pharmaceutical composition comprising the combination as described herein, and at least one pharmaceutically acceptable carrier.

### BRIEF DESCRIPTION OF THE FIGURES

**Figure 1** shows the *in vitro* effect on caspase3.7 activity when combining the FLT3 inhibitor Compound A (Midostaurin), with the Mcl-1 inhibitor Compound B, in AML cell lines Molm13 and MV-4-11.

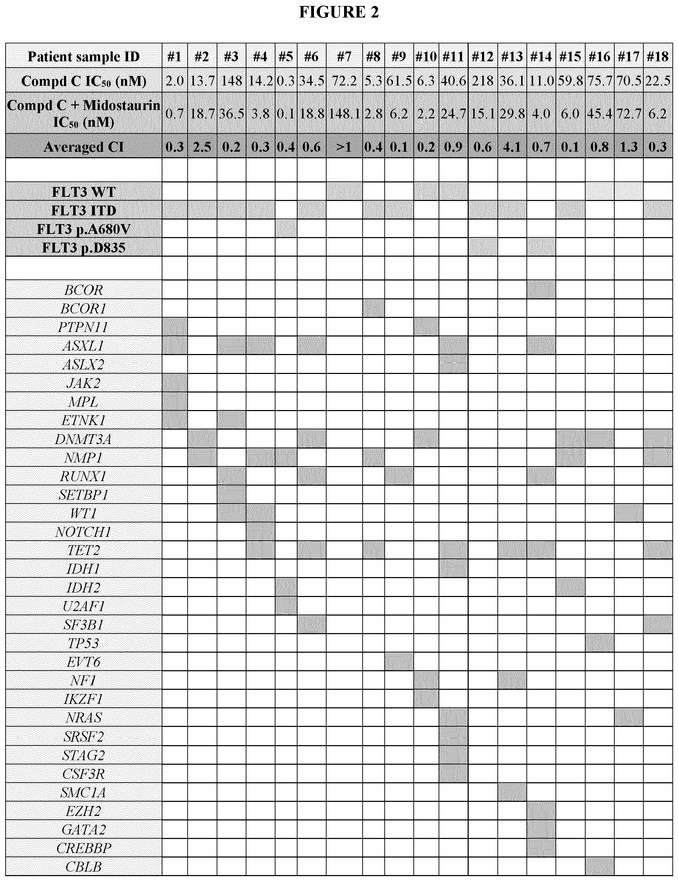

**Figure 2****: Combined targeting of FLT3 and Mcl-1 is efficacious in FLT3-ITD AML primary samples.** Mutation profiling of primary AML samples and sensitivity to combined S63845/Midostaurin treatment for 24 hours. Cell growth was measured by CellTiter-Glo. Combination index (CI) was calculated using CalcuSyn software and is average of CI at ED50, ED75, and ED90. CI <1 indicates synergistic effect and CI >1 antagonism. Primary AML samples were treated with the following serial dilutions of combination doses: Midostaurin, from 3.1 nM to 800 nM; and S63845, from 0.31 nM to 80 nM. Ratio of Midostaurin to S63845 was 10:1. Mutation presence is indicated by filled blue boxes for each sample.
**Figure 3****: Midostaurin induces BIM accumulation which contributes to Midostaurin/S63845 (Compound C) lethality**
   **A.** Midostaurin increased level of pro-apoptotic Bim, which could in turn bind and negate residual Mcl-1 activity. Elevated Bim is sustained upon S63845 co-treatment, suggesting that Bim plays a functional role in Midostaurin/S63845-mediated lethality.
   **B.** Western blot analysis of FLT3-mediated signaling pathways and expression of Bcl-2 family proteins in MV-4-11 cells treated for 6 hours with Midostaurin, S63845 alone or in combination. De-phosphorylation of FLT3-ITD lead to inactivation of STATS and MAPK and was accompanied by significant downregulation of MAPK-mediated phosphorylation of Mcl-1 at Thr163, site required for Mcl-1 stability. Consequently, Midostaurin reduced level of Mcl-1.

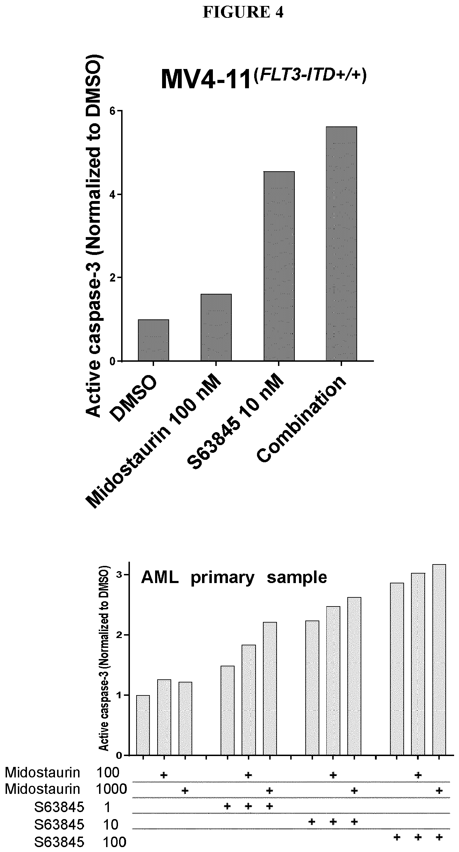

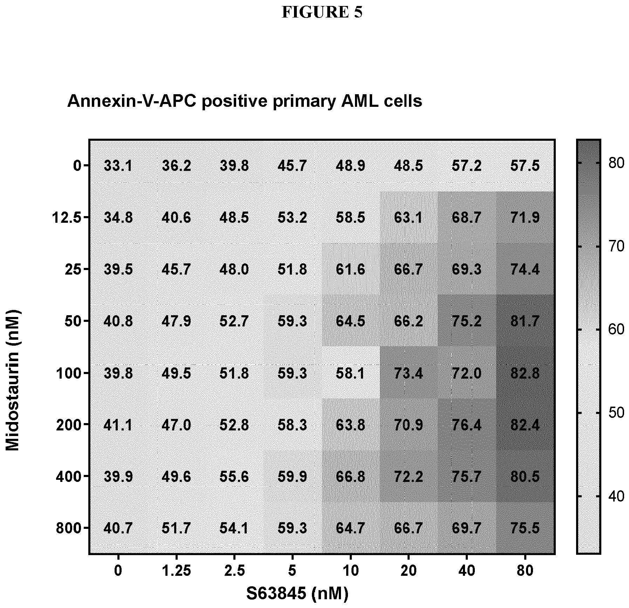

**Figures 4** **and** **5****: Combined targeting of FLT3 and Mcl-1 facilitates apoptosis**
   Caspase activation was measured using Caspase-Glo^{®} 3/7 assay after 6 hours of drug treatment. The percentage of apoptotic cells was measured by flow cytometry using Annexin-V-APC/DAPI staining. AML primary cells were treated for 16 hours.

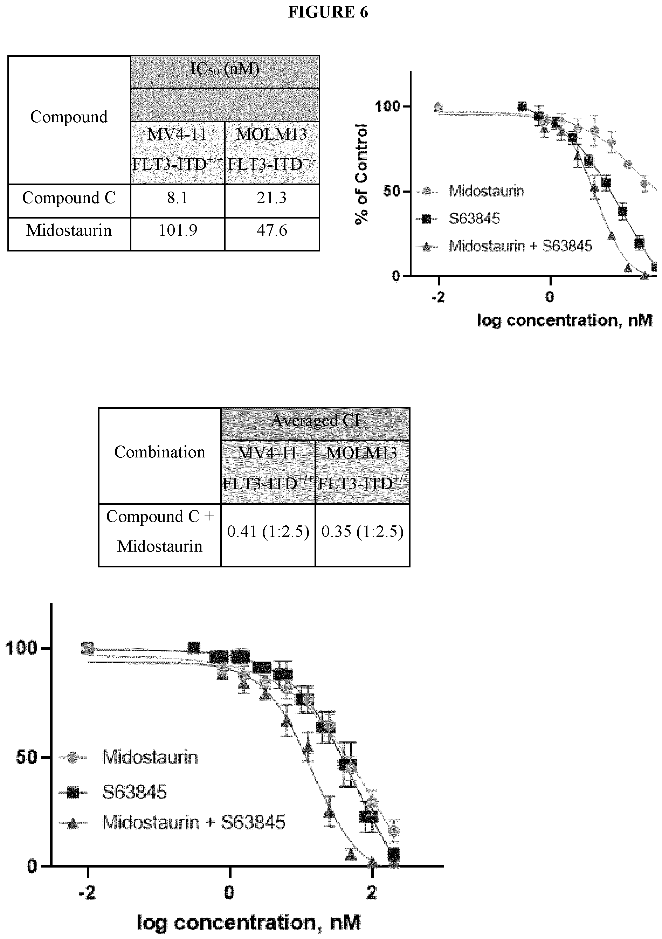

**Figure 6****: Anti-leukemic synergy of S63845 (Compound C) and FLT3 TKi**
   The sensitivity of AML cell lines to S63845 (Compound C) and selected FLT3 inhibitors (FLT3 TKi) after 24 hours exposure. Cell growth was measured by CellTiter-Glo. Combination index (CI) values were calculated using CalcuSyn software and are average of CI at ED50, ED75, and ED90. Ratios at which drugs were combined are shown in brackets.

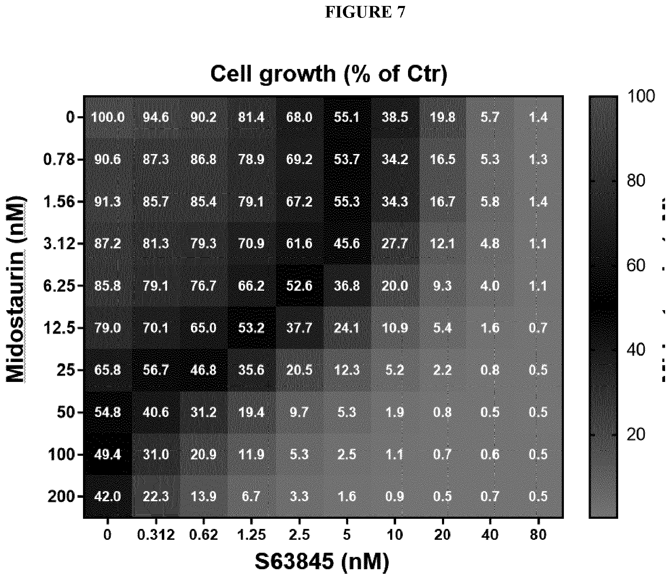

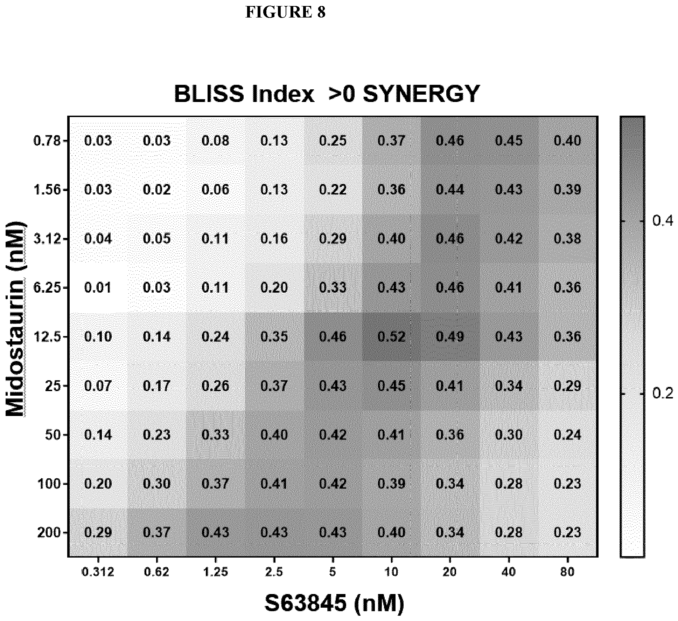

**Figures 7** **and** **8****: Anti-leukemic synergy of S63845 and FLT3 TKi**
   Representative synergistic interactions between S63845 combined with Midostaurin in MV4-11 FLT3-ITD cells determined by large scale drug synergy BLISS Independence model. Cells were treated with nine 2-fold serial dilutions of each compound, either individually or in all possible permutations in a checkerboard fashion. Cell growth was assessed after 24 hours (Figure 7). Synergistic interactions were assessed using BLISS Independence model. BLISS index values for each dose combination >0 represent synergy, whereas BLISS index values <0 represent antagonism (Figure 8).

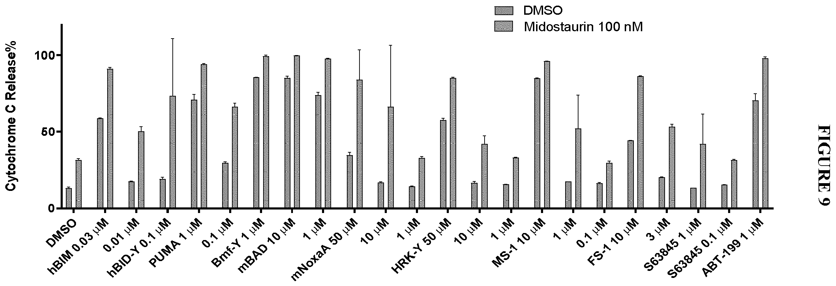

**Figure 9****: BH3 profiling: Midostaurin increases mitochondrial priming for apoptosis in FLT3-ITD cells exposed to S63845**
   BH3 profiling assay shows that Midostaurin increased mitochondrial priming in MV4-11 FLT3-ITD to Mcl-1 inhibitor S63845 and particularly Bim peptide. Cells were subjected to BH3 profiling assay 4 hours after treatment with Midostaurin (100 nM). Increase in cytochrome C release is a quantitative measure of mitochondrial apoptosis initiation.

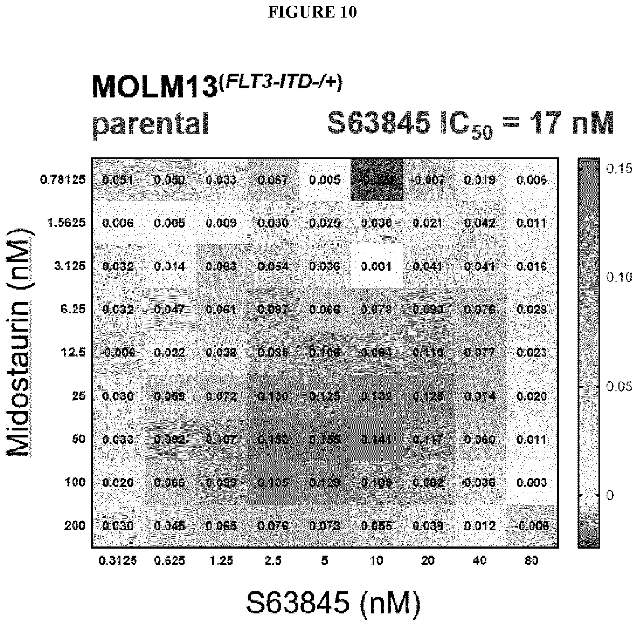

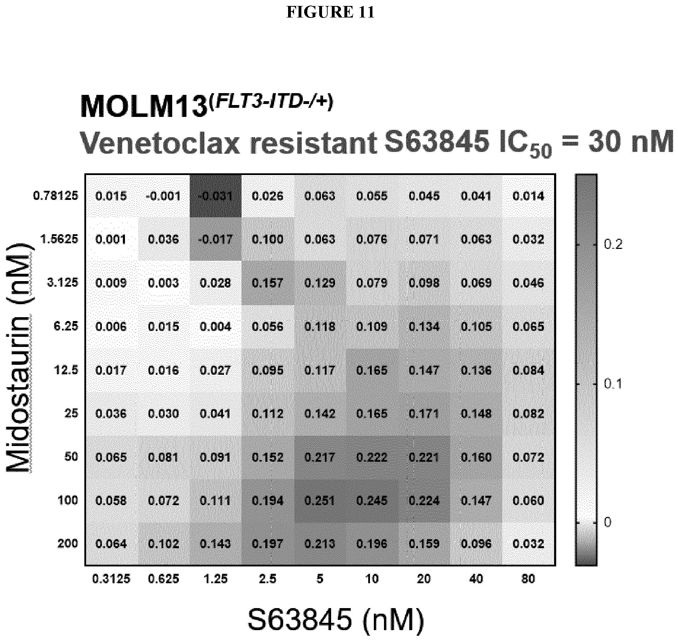

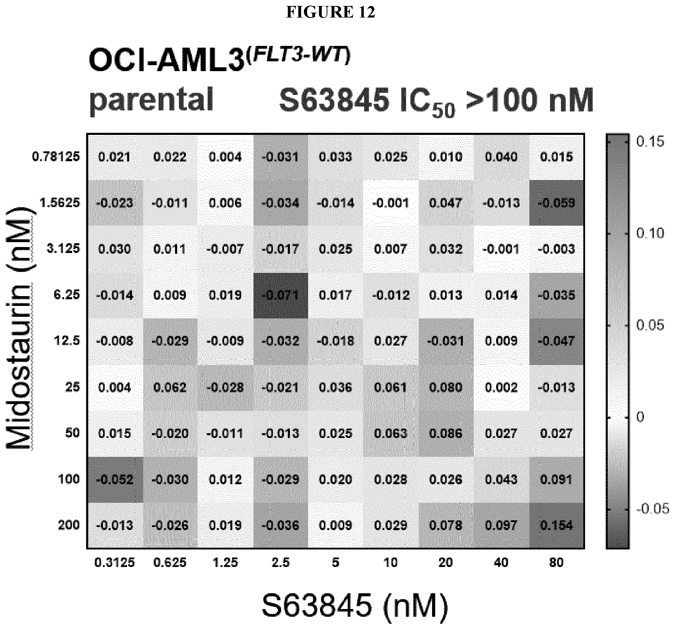

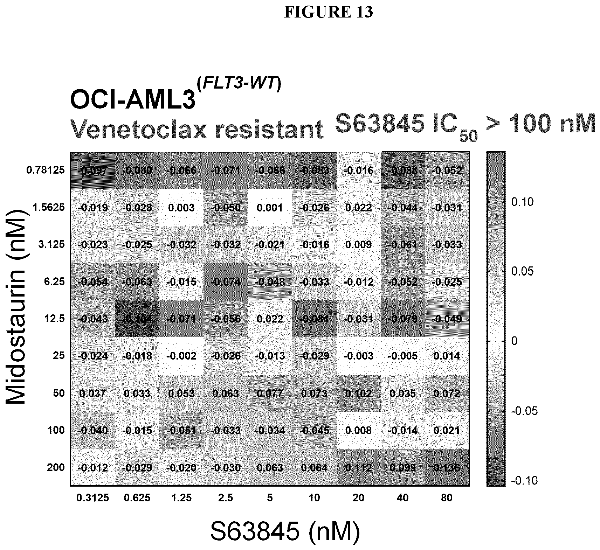

**Figures 10****,** **11****,** **12** **and** **13****: Combination of S63845 and Midostaurin is effective in Venetoclax-resistant AML cells with FLT3-ITD but not in FLT3-WT**
   BLISS Independence model for synergistic interactions between S63845 combined with Midostaurin in MOLM13 FLT3-ITD cells and Venetoclax-resistant subline. Venetoclax-resistant cells were generated as described in Zhang et al. Blood, 2015, 126, 328 and were routinely maintained in medium containing 1 µM Venetoclax. Venetoclax was washed out before experiments and cells were treated with nine 2-fold serial dilutions of S63845 and Midostaurin, either individually or in all possible permutations in a checkerboard fashion. After 24 hours of treatment, cell growth was measured using Cell TiterGlo. BLISS index values for each dose combination >0 represent synergy, whereas BLISS index values <0 represent antagonism.

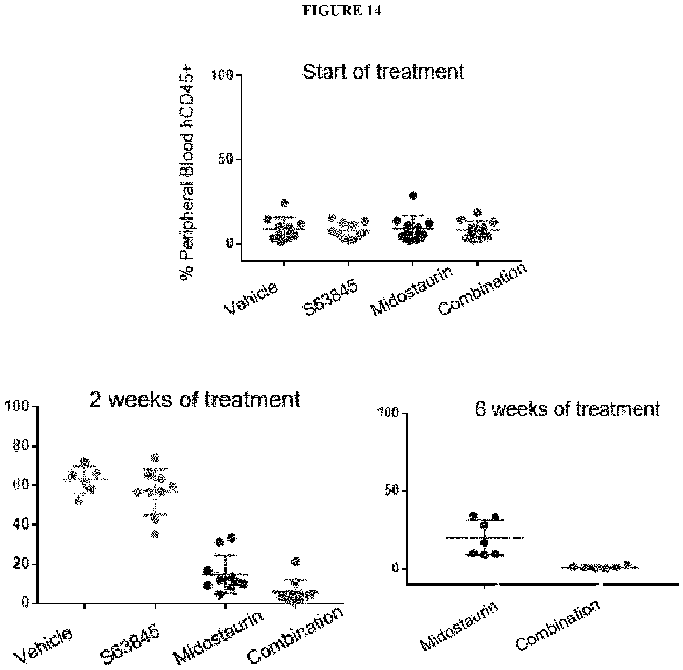

**Figure 14****: Preliminary *in vivo* study: AML FLT3-ITD xenograft**
   NSG mice were transplanted with 10⁶ patient derived AML cells harboring FLT3-ITD mutations. Tumor burden was measured by the percentage of hCD45+ cells in mouse blood using FACS. After engraftment, mice were treated with (i) vehicle, (ii) S63845 40 mg/kg IV once a week, (iii) Midostaurin 75 mg/kg by oral gavage daily, or (iv) combination.

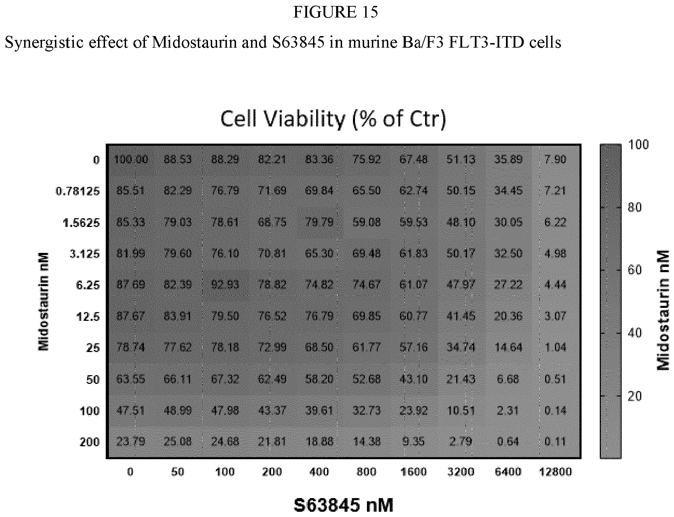

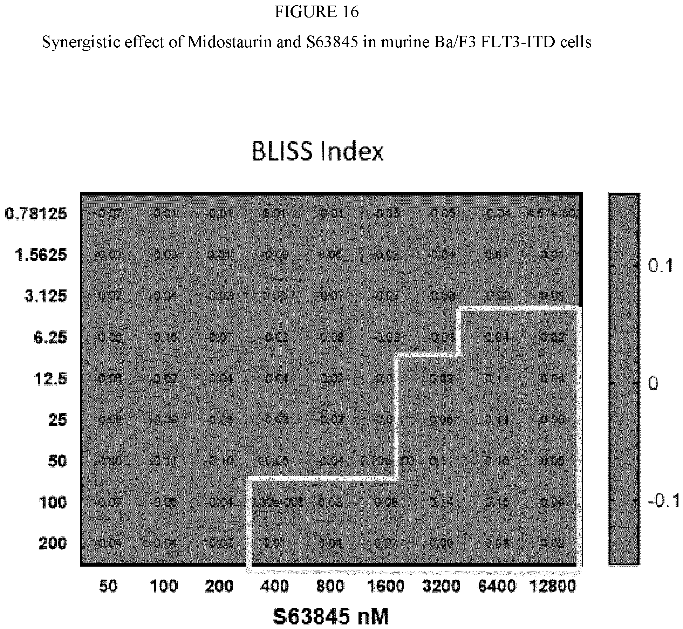

**Figures 15** **and** **16****: Synergistic effect of Midostaurin and S63845 in murine Ba/F3 FLT3-ITD cells**
   Synergistic interactions between S63845 combined with Midostaurin in murine Ba/F3 FLT3-ITD cells determined by large scale drug synergy BLISS Indepencence model. Cells were treated with nine 2-fold serial dilutions of each compound, either individually or in all possible permutations in a checkerboard fashion. Cell growth was assessed after 24 hours (Figure 15). Synergistic interactions were assessed using BLISS Independence model. BLISS index values for each dose combination > 0 represent synergy, whereas BLISS index values < 0 represent antagonism (Figure 16).

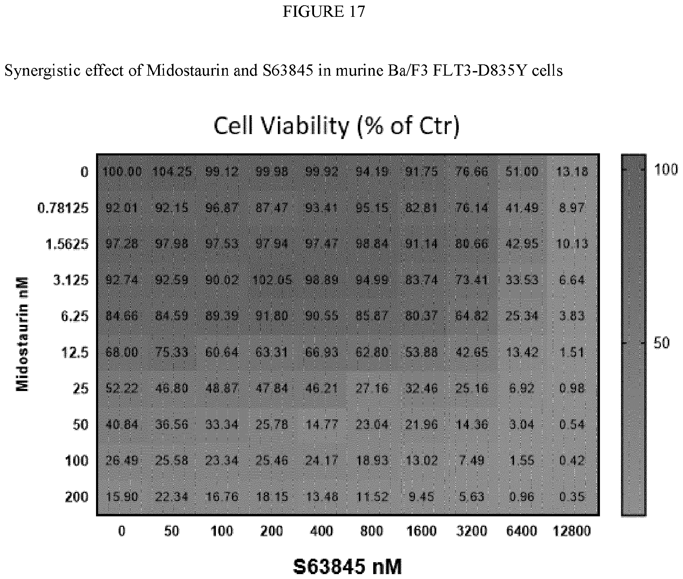

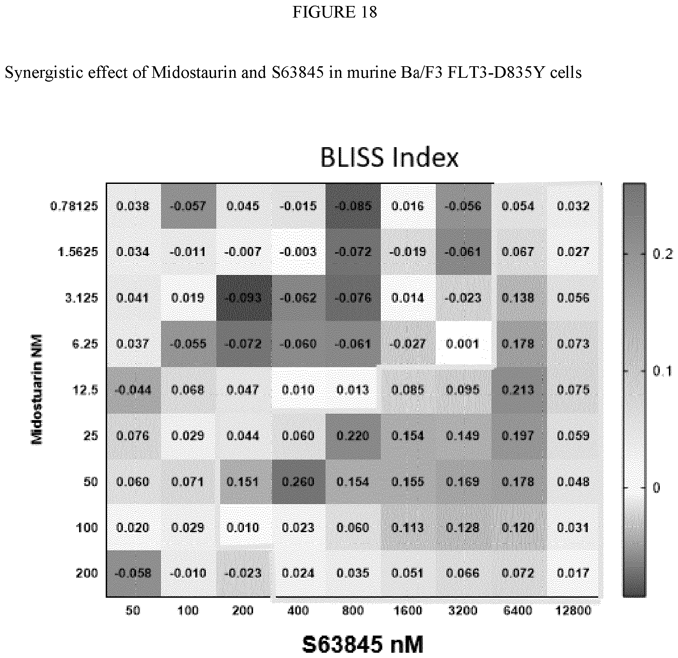

**Figure 17** **and** **18****: Synergistic effect of Midostaurin and S63845 in murine Ba/F3 FLT3-D835Y cells**
   Synergistic interactions between S63845 combined with Midostaurin in murine Ba/F3 FLT3-D835Y cells determined by large scale drug synergy BLISS Indepencence model. Cells were treated with nine 2-fold serial dilutions of each compound, either individually or in all possible permutations in a checkerboard fashion. Cell growth was asseded after 24 hours (Figure 17). Synergistic interactions were assessed using BLISS Independence model. BLISS index values for each dose combination > 0 represent synergy, whereas BLISS index values < 0 represent antagonism (Figure 18).

### DEFINITIONS

"Combination" refers to either a fixed dose combination in one unit dosage form (e.g., capsule, tablet, or sachet), non-fixed dose combination, or a kit of parts for the combined administration where a compound of the present invention and one or more combination partners (e.g., another drug as explained below, also referred to as "therapeutic agent" or "co-agent") may be administered independently at the same time or separately within time intervals, especially where these time intervals allow that the combination partners show a cooperative, e.g., synergistic effect.

The terms "co-administration" or "combined administration" or the like as utilized herein are meant to encompass administration of the selected combination partner to a single subject in need thereof (e.g., a patient), and are intended to include treatment regimens in which the agents are not necessarily administered by the same route of administration or at the same time.

The term "fixed dose combination" means that the active ingredients, e.g., Compound B or Compound C and one or more combination partners, are both administered to a patient simultaneously in the form of a single entity or dosage.

The term "non-fixed dose combination" means that the active ingredients, e.g., a compound of the present invention and one or more combination partners, are both administered to a patient as separate entities either simultaneously or sequentially, with no specific time limits, wherein such administration provides therapeutically effective levels of the two compounds in the body of the patient. The latter also applies to cocktail therapy, e.g., the administration of three or more active ingredients.

"Cancer" means a class of disease in which a group of cells display uncontrolled growth. Cancer types include haematological cancer (lymphoma and leukaemia). In particular "cancer" refers to haematological cancer, in particular acute myeloid leukaemia.

"AML" means acute myeloid leukaemia. In a particular embodiment, the AML is present in patients carrying a *FLT3* mutation. In a further embodiment, the mutation is *FLT3-ITD.* In another embodiment, the mutation is *FLT3-TKD.* In particular, said FLT3-TKD mutation comprises FLT3-D835Y or FLT3-F691 mutation within the tyrosine kinase domain (TKD) of FLT3. The AML to be treated may be resistant to prior anticancer therapy. Such prior therapy may include one or more compounds used to treat AML, for example venetoclax, decitabine, daunorubicin, and cytarabine, in particular venetoclax. In one embodiment, the AML to be treated is venetoclax-resistant AML, especially venetoclax-resistant AML with FLT3-ITD mutation.

The term "jointly therapeutically effective" means that the therapeutic agents may be given separately (in a chronologically staggered manner, especially a sequence-specific manner) in such time intervals that they prefer, in the warm-blooded animal, especially human, to be treated, still show a (preferably synergistic) interaction (joint therapeutic effect). Whether this is the case can, inter alia, be determined by following the blood levels, showing that both compounds are present in the blood of the human to be treated at least during certain time intervals.

"Synergistically effective" or "synergy" means that the therapeutic effect observed following administration of two or more agents is greater than the sum of the therapeutic effects observed following the administration of each single agent.

As used herein, the term "treat", "treating" or "treatment" of any disease or disorder refers in one embodiment, to ameliorating the disease or disorder (i.e., slowing or arresting or reducing the development of the disease or at least one of the clinical symptoms thereof). In another embodiment "treat", "treating" or "treatment" refers to alleviating or ameliorating at least one physical parameter including those which may not be discernible by the patient. In yet another embodiment, "treat", "treating" or "treatment" refers to modulating the disease or disorder, either physically, (e.g., stabilization of a discernible symptom), physiologically, (e.g., stabilization of a physical parameter), or both.

As used herein, a subject is "in need of" a treatment if such subject would benefit biologically, medically or in quality of life from such treatment.

"Medicament" means a pharmaceutical composition, or a combination of several pharmaceutical compositions, which contains one or more active ingredients in the presence of one or more excipients.

### DETAILED DESCRIPTION OF THE INVENTION

Described below are a number of embodiments of the invention, where for convenience E1 is identical to the first aspect of the invention hereinabove. Further enumerated embodiments (E) of the invention are described herein. It will be recognized that features specified in each embodiment may be combined with other specified features to provide further embodiments of the present invention. The present invention is defined by the claims. Any subject-matter falling outside the scope of the claims is provided for information only.

As described below, embodiments E2 to E6, E8, E9, E11, E13, E15 to E18, and E21 to E26 of the invention are claimed and form part of the invention. Embodiments E7, E10, E12, E14, E19 and E20 represent further aspects according to the invention.

E2. The combination according to E1, wherein the Mcl-1 inhibitor is Compound B: (2*R*)-2-{[(5*Sₐ*)-5-{3-chloro-2-methyl-4-[2-(4-methylpiperazin-1-yl)ethoxy]phenyl}-6-(4-fluorophenyl)thieno[2,3-*d*]pyrimidin-4-yl]oxy}-3-(2-{[2-(2-methoxyphenyl)pyrimidin-4-yl]methoxy}phenyl)propanoic acid, or a pharmaceutically acceptable salt thereof.

E3. The combination according to E1, wherein the Mcl-1 inhibitor is Compound C: (2*R*)-2-{[(5*Sₐ*)-5-{3-chloro-2-methyl-4-[2-(4-methylpiperazin-1-yl)ethoxy]phenyl}-6-(5-fluorofuran-2-yl)thieno[2,3-*d*]pyrimidin-4-yl]oxy}-3-(2-{[1-(2,2,2-trifluoroethyl)-1*H-*pyrazol-5-yl]methoxy}phenyl)propanoic acid, or a pharmaceutically acceptable salt thereof.

E4. The combination according to E2, wherein Compound B is administered intravenously and Midostaurin is administered orally.

E5. The combination according to any of E1 to E4, further comprising at least one additional anti-cancer agent, for example, cytarabine and / or daunorubicin.

E6. The combination according to any of E1 to E5, in the form of a non-fixed dose combination.

E7. The combination according to any of E1 to E5, in the form of a fixed dose combination.

E8. The combination according to any of E1 to E7, for use in medicine.

E9. The combination according to any of E1 to E7, for use according to E8, wherein said use is in the treatment of cancer.

E10. The combination according to any of E1 to E7, for use according to E9, wherein the cancer is haematological cancer.

E11. The combination according to any of E1 to E7, for use according to E9, wherein the cancer is acute myeloid leukaemia. In particular, the acute myeloid leukaemia is present in patients carrying a *FLT3* mutation. More particularly, said mutation is *FLT3-ITD.*

E12. The combination according to any of E2 or E4 to E7, for use according to any of E8 to E11, wherein Midostaurin and Compound B are provided in amounts which are jointly therapeutically effective for the treatment of cancer.

E13. The combination according to any of E2 or E4 to E7, for use according to any of E8 to E11, wherein Midostaurin and Compound B are provided in amounts which are synergistically effective for the treatment of cancer.

E14. The combination according to any of E2 or E4 to E7, for use according to any of E8 to E11, wherein Midostaurin and Compound B are provided in synergistically effective amounts which enable a reduction of the dose required for each compound in the treatment of cancer, whilst providing an efficacious cancer treatment, with a reduction in side effects.

E15. A pharmaceutical composition comprising the combination according to any of E1 to E7, and at least one pharmaceutically acceptable carrier.

E16. The use of a combination according to any of E1 to E7, in the manufacture of a medicament for the treatment of cancer.

E17. The use according to E16, of a combination according to any of E1 to E7, wherein the cancer is acute myeloid leukaemia. In particular, the acute myeloid leukaemia is present in patients carrying a *FLT3* mutation. More particularly, said mutation is *FLT3-ITD* or *FLT3-TKD.*

E18. A medicament containing, separately or together,
(a) Midostaurin, or a pharmaceutically acceptable salt thereof, or a complex thereof, or a co-crystal thereof, or a solvate, including hydrate, thereof, and
(b) Compound B: (2*R*)-2-{[(5*Sₐ*)-5-{3-chloro-2-methyl-4-[2-(4-methylpiperazin-1-yl) ethoxy]phenyl}-6-(4-fluorophenyl)thieno[2,3-*d*]pyrimidin-4-yl]oxy}-3-(2-{[2-(2-methoxyphenyl)pyrimidin-4-yl]methoxy}phenyl)propanoic acid, or a pharmaceutically acceptable salt thereof,
for simultaneous, sequential or separate administration, and wherein Midostaurin and Compound B are provided in effective amounts for the treatment of cancer, in particular AML.

E19. Compound B for use in a combination therapy with Midostaurin, for the treatment of cancer, in particular AML.

E20. Midostaurin for use in a combination therapy with Compound B, for the treatment of cancer, in particular AML.

E21. The combination for use, or use as described in any one of the embodiments herein, for the treatment of AML resistant to prior therapy.

E22. The combination for use, or use as described in any one of the embodiments herein, for the treatment of AML resistant to one or more compounds selected from venetoclax, decitabine, daunorubicin, and cytarabine, in particular venetoclax.

E23. The combination for use, or use as described in any one of the embodiments herein, for the treatment of venetoclax-resistant AML with *FLT3-ITD* mutation.

E24. The combination for use, or use as described in any one of the embodiments herein, for the treatment of AML with *FLT3-TKD* mutation.

E25. The combination for use, or use as described in embodiment E22, wherein said *FLT3-TKD* mutation comprises FLT3-D835Y or FLT3-F691 mutation within the tyrosine kinase domain (TKD) of FLT3.

E26. The combination for use, or use as described herein, wherein the cancer is acute myeloid leukaemia present in patients carrying a FLT3-ITD mutation in the presence of a FLT3-TKD mutation.

In another aspect, the invention provides a combination, combination for use, composition, medicament or use, as described herein, in particular comprising Compound B or Compound C and Midostaurin, wherein said cancer, in particular AML, is resistant to prior anticancer therapy. Such prior therapy may include one or more compounds used to treat cancer such as AML, for example venetoclax, decitabine, daunorubicin, and cytarabine, in particular venetoclax. In one embodiment, the AML to be treated is Venetoclax-resistant AML, especially Venetoclax-resistant AML with FLT3-ITD.

In another embodiment, the AML to be treated is AML with FLT3-TKD mutation. In particular, said FLT3-TKD mutation comprises FLT3-D835Y or FLT3-F691 mutations within tyrosine kinase domain (TKD) of FLT3. In a further embodiment, the cancer is acute myeloid leukaemia present in patients carrying a *FLT3-ITD* mutation in the presence of a FLT3-TKD mutation.

Particularly, the AML to be treated as described herein is resistant to FLT3 inhibitor treatment.

In the pharmaceutical compositions according to the invention, the proportion of active ingredients by weight (weight of active ingredients over the total weight of the composition) is from 5 to 50 %.

Among the pharmaceutical compositions according to the invention there will be more especially used those which are suitable for administration by the oral, parenteral and especially intravenous, per- or trans-cutaneous, nasal, rectal, perlingual, ocular or respiratory route, more specifically tablets, dragées, sublingual tablets, hard gelatin capsules, glossettes, capsules, lozenges, injectable preparations, aerosols, eye or nose drops, suppositories, creams, ointments, dermal gels etc.

In one embodiment, Compound B is administered intravenously, for example, using the formulation as described in WO 2018/078064.

The pharmaceutical compositions according to the invention comprise one or more excipients or carriers selected from diluents, lubricants, binders, disintegration agents, stabilisers, preservatives, absorbents, colourants, sweeteners, flavourings etc.

### By way of non-limiting example there may be mentioned:

◆ *as diluents*: lactose, dextrose, sucrose, mannitol, sorbitol, cellulose, glycerol,
◆ *as lubricants*: silica, talc, stearic acid and its magnesium and calcium salts, polyethylene glycol,
◆ *as binders*: magnesium aluminium silicate, starch, gelatin, tragacanth, methylcellulose, sodium carboxymethylcellulose and polyvinylpyrrolidone,
◆ *as disintegrants*: agar, alginic acid and its sodium salt, effervescent mixtures.

The compounds of the combination may be administered simultaneously, separately or sequentially. The corresponding pharmaceutical compositions may allow the instantaneous or delayed release of the active ingredients. The compounds of the combination may moreover be administered in the form of two separate pharmaceutical compositions, each containing one of the active ingredients, or in the form of a single pharmaceutical composition, in which the active ingredients are in admixture.

The useful dosage regimen varies according to the sex, age and weight of the patient, the administration route, the nature of the cancer and of any associated treatments and ranges from 25 mg to 1500 mg of Mcl-1 inhibitor per week, more preferably from 50 mg to 1400 mg per week.

The dosage of Midostaurin for the treatment of AML that is *FLT3* mutation positive is 50 mg orally, twice daily with food. Midostaurin is provided to the patient in combination with standard cytarabine and daunorubicin induction and cytarabine consolidation. This dose may be adapted as necessary in the combination treatment with Compound B.

In one embodiment, oral administration of Midostaurin is by solid form. The dosage form of Midostaurin is a soft gelatin capsule with 25 mg of drug substance.

### EXAMPLE 1: The in vitro effect on caspase3.7 activity when combining the FLT3 inhibitor Compound A (Midostaurin) with the Mcl-1 inhibitor Compound B, in AML cell lines Molm13 and MV-4-11.

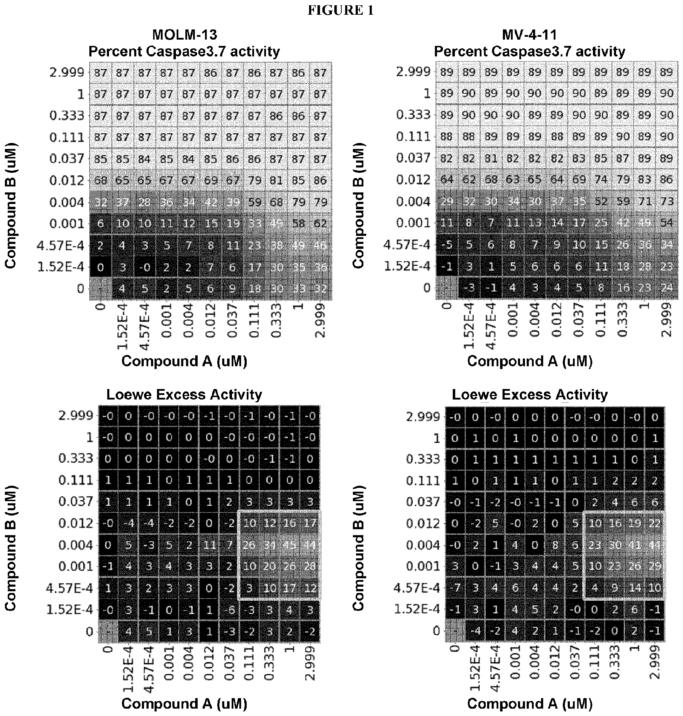

We assessed Mcl-1 and FLT3 inhibitors as single agents and in combination for their ability to induce apoptosis (i.e. Caspase3.7 activation) in leukemic cells. We utilized two AML cell lines with FLT3-ITD mutation (Molm13 and MV-4-11). We tested ability of Compounds A and B to activate caspase3.7 in combinations and as single agents. Compound A as a single agent caused induction of caspase activity with maximum increase of 33 % in Molm13 and 24 % in MV-4-11. Compound B also induced caspase activity with a max increase of 87 % in Molm13 and 89 % in MV-4-11. When the two compounds were combined, a synergistic induction of caspase3.7 activity was observed. The synergy was observed in both cell lines with the synergy score of 3.1 in both. Strong synergy was evident particularly at the lower doses of Compound B. Results are shown in Figure 1.

### Methods

Both compounds were dissolved in 100 % DMSO (Sigma, Catalog number D2650) at concentrations of 10 mM and stored at -20 °C until use.

The cell line Molm13 used in this study was purchased from Leibniz-Institut DSMZ (ACC 554) and MV-4-11 from American Type Cell Collection (ATCC^{®} CRL-9591). The cell lines were cultured at 37 °C in a 5 % CO₂ incubator, Molm13 in RPMI (Lonza, 12-702F) and MV-4-11 in INMM (Hyclone, SH30228.01) media complemented with 10 % fetal bovine serum (Seradigm, 1500-500). The cells were passaged twice a week and the medium was changed every 2 to 3 days.

Caspase3.7 activity was measured by the Caspase-Glo^{®} 3/7 kit from Promega (G8092). 9000 cells/well were dispensed into clear-bottom 384-well black plates (Greiner, #781091) in triplicates with 30 µl/well growth media. 10 ul/well compound mix of 4X was added to each well on 384 plates for 6 hours treatment. Then 30 ul/well of the Caspase-Glo^{®} 3/7 reagents was added to each well at the end and luminescence was recorded on an Envision plate reader (Perkin Elmer). Measured Luminescence is proportional to the amount of caspase activity present.

To evaluate the caspase3.7 activity of the combination in a non-biased way, as well as to identify synergistic effect at all possible concentrations, the studies were conducted with a "dose matrix." This utilized all possible permutations of serially-diluted Compound A and Compound B.

The Compound A and Compound B "dose matrix", consisted of the following: both compounds were subjected to a 11 dose 1:3 serial dilution with the highest dose of 3 uM and down to no-compound control. In the combination assays, agents were applied simultaneously. Luminescent signal values for each single agent and combination treatment were compared to that of untreated controls. The percent caspase3.7 activity was calculated using the following calculation: (1-control/compound treated) x 100. The values for percent of caspase3.7 activity of all wells were calculated using the Chalice software (CombinatoRx, Cambridge MA) and Chalice Analyser as described in Lehar et al., Nature Biotechnology 2009, 27(7), 659-66. In this study, caspase3.7 activity data were analysed instead of cell growth inhibition data with the same mathematical protocols. The caspase3.7 activity relative to control is displayed in the panel labelled "Percent Casapse3.7 Activity", and the amount of activity in excess of the expected amount in the panel labelled "Loewe Excess Activity"). Concentrations of Compound A are shown along the bottom row from left to right and increasing concentrations of Compound B along the leftmost column from bottom to top. All remaining points in the grids display results from a combination of the two inhibitors that correspond to the single agent concentrations denoted on the two axes. Data analysis of caspase3.7 activity was performed using Chalice Analyser. Excess activity was calculated using the Loewe synergy model which measures the effect on caspase3.7 activity to what would be expected if two drugs behave in a dose additive manner. Positive numbers represent areas of increasing synergy. The interpretations of synergy scores are following: SS ~ 0 = Dose Additive, SS >1 = Weak Synergy and SS >2 = Synergy.

### EXAMPLE 2: Combination of Mcl-1 inhibitor Compound C and Midostaurin is synergistic in FLT3-ITD mutated AML cells including those resistant to Bcl-2 inhibitor Venetoclax and in primary AML cells

### Materials and methods

### Figure 2: Combined targeting of FLT3 and Mcl-1 is efficacious in FLT3-ITD AML primary samples. Cell viability assay and Combination index (CI)

Primary AML cells were obtained from peripheral blood draw collected from patients at M. D. Anderson Cancer Center with newly diagnosed or recurrent AML and a high (> 40 %) blast count. Following Ficoll purification AML blasts (8 x 10⁵/well) were seeded in 96-well plates in 100 µL of complete RPMI medium containing 10% FBS (Sigma) and 1x Pen/Strep (Sigma). Midostaurin and S63845 were prepared as 10 mM stocks in DMSO and kept in -80 °C before analysis. Each drug was diluted in complete RPMI medium and given as 4x concentrated solution prepared in 50 µL medium. Control cells received 100 µL of medium containing DMSO (volume of DMSO corresponded to sum of volumes of Midostaurin and S63845 stocks used to make 4x solutions). Cells were incubated with drugs used alone or in combination for 24 hours. Cell viability was measured using CellTiter-Glo Luminescence assay (Promega) according to the manufacturer's instructions. Briefly, cells were gently mixed by pipetting and 35 µL of cell suspension was transferred to white opaque 96-well plates. Next, 80 µL of CellTiter-Glo reagent diluted at 1:3 in PBS was added to each well and cells were incubated for 30 minutes in dark on a plate shaker. Luminescence was read using Tekan plate reader (Infinite m200 pro). The analysis of the combined effects of drugs was done using the combination index (CI) calculated by CalcuSyn (BioSoft, Ferguson, MO, USA) software. A CI of < 1, 1 and > 1 indicates synergism, an additive effect and antagonism, respectively.

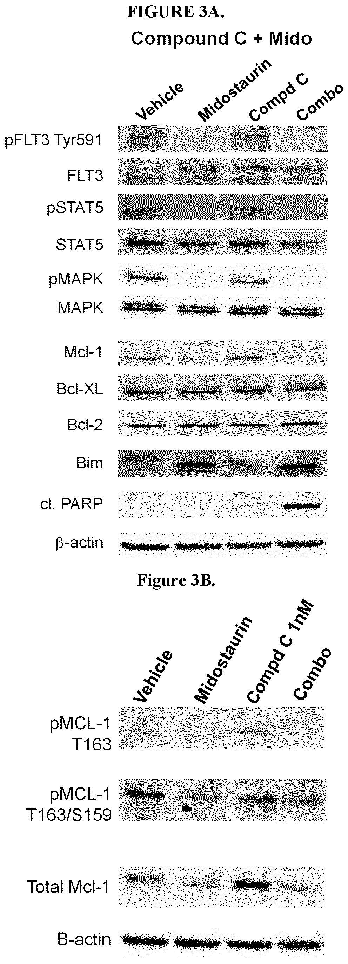

### Figures 3A/3B: Midostaurin induces BIM accumulation which contributes to Midostaurin/S63845 (Compound C) lethality. Western blot analysis

Cells (3 × 10⁶/well) were seeded in 6-well plates in 5 mL of complete RPMI medium and exposed to Midostaurin and S63845 given alone or in combination. Control cells received DMSO. Cells were incubated for 6 hours and then collected, washed twice in PBS and lysed in RIPA buffer (ThermoFisher Scientific) supplemented with 1x Protease and Phosphatase Inhibitor cocktail (ThermoFisher Scientific). Lysates were kept on ice for 20 minutes with vortexing every 5 minutes, then briefly sonicated and centrifuged at 13500 rpm for 15 minutes. Supernatant was collected and protein content was measured using BSA assay. Cell lysates (30-50 µg protein per well) were resolved by electrophoresis on 4 %-20 % gradient precast sodium dodecyl sulfate-polyacrylamide gels (Bio-Rad, Hercules, CA) and transferred to PVDF membrane (Bio-Rad). The membranes were first incubated in Odyssey Blocking Buffer (Li-Cor, Lincon, Nebraska, USA) for 1 hour to block nonspecific protein binding, then with primary antibody overnight at 4 °C, washed with TBST three times, and incubated with IRDye-conjugated secondary antibody (1: 15000 dilution) for 1 hour at room temperature. Membranes were scanned using Odyssay Imaging System (Li-Cor).

### Figure 4: Combined targeting of FLT3 and Mcl-1 facilitates apoptosis. Caspase 3/7 activity assay for apoptosis detection

AML cells or AML primary blasts (3 x 10⁶/well) were seeded in 6-well plates in 5 mL of complete RPMI medium and exposed to midostaurin and S63845 given alone or in combination. Control cells received DMSO. Cells were incubated for 6 hours and then 100 µL of cell suspension was transferred into white opaque 96-well plate, mixed with 100 µL of Caspase-Glo3/7 Reagent (Promega) and incubated in dark for 30 minutes on plate shaker at room temperature. Luminescence was read using Tekan plate reader (Infinite m200 pro). Changes in caspase-3/7 activity following drugs treatment were normalized to DMSO alone. Remaining cells were used for western blot analysis.

### Figure 5: Combined targeting of FLT3 and Mcl-1 facilitates apoptosis. Annexin-V/DAPI assay

AML blasts (8 x 10⁵/well) were seeded in round bottom 96-well plates in 100 µL of complete RPMI medium containing 10% FBS (Sigma) and 1x Pen/Strep (Sigma). Midostaurin and S63845 were diluted in complete RPMI medium and given as 4x concentrated solution prepared in 50 µL medium. Control cells received 100 µL of medium containing DMSO (volume of DMSO corresponded to sum of volumes of Midostaurin and S63845 stocks used to make 4x solutions). Cells were incubated with drugs given alone or in combination for 24 hours. Next day, plates with cells were centrifuged at 1500 rpm for 5 minutes. Then, cells were washed once in PBS and resuspended in 100 µL of Annexin-V binding buffer containing 0.3 µL of Annexin-V-APC (BD Bioscience) and 5 µL of DAPI (2 µg/mL). Cells were stained in dark for 20 minutes, supplemented with additional 100 µL of Annexin-V binding buffer and analyzed by BD LRSII flow cytometer (Becton Dickinson). Annexin-V-APC positive cells were identified using FlowJo software (LLC, Ashland, Oregon, USA).

### Figure 6: Anti-leukemic synergy of S63845 (Compound C) and FLT3 TKi. Cell viability assay

MV4-11 FLT3-ITD^{+/+} and MOLM13 FLT3-ITD^{+/-} AML cells (8 x 10⁵/well) were seeded in 96-well plates in 100 µL of complete RPMI medium containing 10 % FBS (Sigma) and 1x Pen/Strep (Sigma). Midostaurin and S63845 were diluted in complete RPMI medium and given as 4x concentrated solution prepared in 50 µL medium. Control cells received 100 µL of medium containing DMSO (volume of DMSO corresponded to sum of volumes of Midostaurin and S63845 stocks used to make 4x solutions). Cells were incubated with drugs given alone or in combination for 24 hours. Cell viability was measured using CellTiter-Glo Luminescence assay (Promega) according to the manufacturer's instructions. Briefly, cells were gently mixed by pipetting and 35 µL of cell suspension was transferred to white opaque 96-well plates. Next, 80 µL of CellTiter-Glo reagent diluted at 1:3 in PBS was added to each well and cells were incubated for 30 minutes in dark on a plate shaker. Luminescence was read using Tekan plate reader (Infinite m200 pro). The analysis of the combined effects of drugs was done using the combination index (CI) calculated by CalcuSyn (BioSoft, Ferguson, MO, USA) software. A CI of < 1, 1 and > 1 indicates synergism, an additive effect and antagonism, respectively.

### Figures 7 and 8: Anti-leukemic synergy of S63845 and FLT3 TKi. Cell viability assay and BLISS index

MV4-11 FLT3-ITD^{+/+} cells (8 × 10⁵/well) were seeded in 96-well plates in 100 µL of complete RPMI medium containing 10 % FBS (Sigma) and 1x Pen/Strep (Sigma). Midostaurin and S63845 were diluted in complete RPMI medium and given as 4x concentrated solution prepared in 50 µL medium. Cells were treated for 24 hours with nine 2-fold serial dilutions of each compound, either individually or in all possible permutations in a checkerboard fashion. Cell growth was measured viability was measured using CellTiter-Glo Luminescence assay (Promega) according to the manufacturer's instructions. Briefly, cells were gently mixed by pipetting and 35 µL of cell suspension was transferred to white opaque 96-well plates. Next, 80 µL of CellTiter-Glo reagent diluted at 1:3 in PBS was added to each well and cells were incubated for 30 minutes in dark on a plate shaker. Luminescence was read using Tekan plate reader (Infinite m200 pro). Cell growth was expressed as % of DMSO treated cells. Synergistic interactions were assessed using BLISS Independence model. BLISS index values for each dose combination > 0 represent synergy, whereas BLISS index values < 0 represent antagonism.

### Figure 9: Midostaurin increases mitochondrial priming for apoptosis in FLT3-ITD cells exposed to S63845. Dynamic BH3 profiling

BH3 profiling was performed in black 384-well plates according to A. Letai method (https://letailab.dana-farber.org/bh3-profiling.html). Briefly, cells (3 × 10⁶/well) were seeded in 6-well plates in 5 mL of complete RPMI medium and exposed to Midostaurin and S63845 given alone or in combination. Control cells received DMSO. Cells were incubated for 6 hours, collected, washed twice in PBS and resuspended in MEB buffer (150 mM mannitol, 10 mM HEPES-KOH pH 7.5, 150 mM KCl, 1 mM EGTA, 1 mM EDTA, 0.1 % BSA, 5 mM succinate) to a final density 1 × 10⁶/mL. Cells (15 µL) were added to 384-well plate containing containing peptides/BH3 mimetics and 15 µL of 0.002 % digitonin in MEB buffer. Cells were incubated for 1 hour in 25 °C and subsequently fixed in 10 µL of 4 % formaldehyde in PBS for 10 minutes. To terminate the fixation, 10 µL of N2 buffer (1.7M Tris, 1.25M Glycine pH 9.1) was added and cells were left at room temperature for 5 minutes. Then, cells were mixed with 10 µL of 10x CytoC Stain Buffer (10 % BSA, 2 % Tween20 in PBS) containing anti-Cytochrome C antibody (Clone 6H2.B4 labeled with a fluorescent tag, BioLegend) at 1:40 dilution and stained overnight at 4 °C. Cytochrome C release was measured by flow cytometry.

### Figures 10, 11, 12 and 13: Combination of S63845 and Midostaurin is effective in Venetoclax-resistant AML cells with FLT3-ITD but not in FLT3-WT. Cell viability assay and BLISS index in venetoclax resistant AML cells

Venetoclax-resistant cell lines (MOLM13 FLT3-ITD^{+/-} and OCI-AML3 FLT3-WT), were generated by exposing the cells to gradually increasing concentrations of venetoclax. Cells were routinely maintained in complete RPMI medium containing 1 µM of venetoclax. 24 hours before testing the effect of combination of Midostaurin and S63845 on cell viability, cells were cultured in venetoclax-free RPMI medium. Venetoclax resistant cells and their parental counterparts were subsequently seeded in 96-well plates in 100 µL of complete RPMI medium containing 10 % FBS (Sigma) and 1x Pen/Strep (Sigma). Midostaurin and S63845 were diluted in complete RPMI medium and given as 4x concentrated solution prepared in 50 µL medium. Cells were treated for 24 hours with nine 2-fold serial dilutions of each compound, either individually or in all possible permutations in a checkerboard fashion. Cell growth was measured viability was measured using CellTiter-Glo Luminescence assay (Promega) according to the manufacturer's instructions. Briefly, cells were gently mixed by pipetting and 35 µL of cell suspension was transferred to white opaque 96-well plates. Next, 80 µL of CellTiter-Glo reagent diluted at 1:3 in PBS was added to each well and cells were incubated for 30 minutes in dark on a plate shaker. Luminescence was read using Tekan plate reader (Infinite m200 pro). Cell growth was expressed as % of DMSO treated cells. Synergistic interactions were assessed using BLISS Independence model. BLISS index values for each dose combination > 0 represent synergy, whereas BLISS index values < 0 represent antagonism.

### Figure 14: Preliminary in vivo study: AML FLT3-ITD xenograft. AML-PDX model

For the AML-PDX model, female NSG mice (6 weeks of age, The Jackson Laboratory) were irradiated with 200 cGy and i.v. injected with AML-PDX (1 × 10⁶ cells/100 µl). Peripheral blood was collected once a week via the retro-orbital route and processed to measure hCD45⁺ cells by flow cytometry to confirm the establishment of leukemia. When leukemia engraftment reached 2-10 %, mice were grouped for treatment with vehicle, Midostaurin (75 mg/kg, daily oral gavage), S63845 (40 mg/kg i.v. once a week) or combination. Once every two weeks peripheral blood was collected through the retro-orbital route and processed to measure leukemia burden (hCD45⁺ cells) by flow cytometry.

The experiments of Figures 2-14 show that combination of Mcl-1 inhibitor Compound C and Midostaurin is synergistic in FLT3-ITD mutated AML cells including those resistant to Bcl-2 inhibitor Venetoclax and in primary AML cells. Elevated Bim levels and increased mitochondrial priming in response to hBIM peptide following Midostaurin exposure suggests that Bim may play a functional role in Midostaurin/S63845-mediated lethality.

### EXAMPLE 3: Cell viability assay and BLISS index for Ba/F3 FLT3-ITD and FLT3-D835Y cells

Murine Ba/F3 FLT3-ITD and FLT3-D835Y cells (8 x 10⁵/well) were seeded in 96-well plates in 100 µL of complete RPMI medium containing 10 % FBS (Sigma) and 1x Pen/Strep (Sigma). Midostaurin and S63845 were diluted in complete RPMI medium and given as 4x concentrated solution prepared in 50 µL medium. Control cells received 100 µL of medium containing DMSO (volume of DMSO corresponded to sum of volumes of Midostaurin and S63845 stocks used to make 4x solutions). Cells were incubated with drugs given alone or in combination for 24 hours. Cell viability was measured using CellTiter-Glo Luminescence assay (Promega) according to the manufacturer's instructions. Briefly, cells were gently mixed by pipetting and 35 µL of cell suspension was transferred to white opaque 96-well plates. Next, 80 µL of CellTiter-Glo reagent diluted at 1:3 in PBS was added to each well and cells were incubated for 30 minutes in dark on a plate shaker. Luminescence was read using Tekan plate reader (Infinite m200 pro). The analysis of the combined effects of drugs was done using the combination index (CI) calculated by CalcuSyn (BioSoft, Ferguson, MO, USA) software. A CI of < 1, 1 and > 1 indicates synergism, an additive effect and antagonism, respectively.

### Results

Figures 15 and 16 show synergistic interactions between S63845 and Midostaurin in murine Ba/F3 FLT3-ITD cells determined by large scale drug synergy BLISS Independence model. Cells were treated with nine 2-fold serial dilutions of each compound, either individually or in all possible permutations in a checkerboard fashion. Cell growth was assessed after 24 hours (heat map, left panel). BLISS index values for each dose combination > 0 represent synergy, whereas BLISS index values < 0 represent antagonism.

Figures 17 and 18 show synergistic interactions between S63845 and Midostaurin in murine Ba/F3 FLT3-D835Y cells determined by large scale drug synergy BLISS Independence model. Cells were treated with nine 2-fold serial dilutions of each compound, either individually or in all possible permutations in a checkerboard fashion. Cell growth was asseded after 24 hours (heat map, left panel). BLISS index values for each dose combination > 0 represent synergy, whereas BLISS index values < 0 represent antagonism.

S63845 and Midostaurin combination elicited marked synergy in murine Ba/F3 cells expressing either FLT3-ITD or FLT3-D835Y point mutations within tyrosine kinase domain (TKD) of FLT3. The latter observation suggests that S63845/Midostaurin combination may provide substantial efficacy in FLT3-ITD mutants even in the presence of the FLT3 TKD mutations that have been clinically shown to confer resistance to FLT3 inhibitor treatment.

## Claims

1. A combination of
(a) Midostaurin, or a pharmaceutically acceptable salt thereof, or a complex thereof, or a co-crystal thereof, or a solvate, including hydrate, thereof, and
(b) a Mcl-1 inhibitor which is Compound B: (2*R*)-2-{[(5*Sₐ*)-5-{3-chloro-2-methyl-4-[2-(4-methylpiperazin-1-yl)ethoxy]phenyl}-6-(4-fluorophenyl)thieno[2,3-*d*]pyrimidin-4-yl]oxy}-3-(2-{[2-(2-methoxyphenyl)pyrimidin-4-yl]methoxy}phenyl)propanoic acid, or Compound C: (2*R*)-2-{[(5*Sₐ*)-5-{3-chloro-2-methyl-4-[2-(4-methylpiperazin-1-yl)ethoxy]phenyl}-6-(5-fluorofuran-2-yl)thieno[2,3-*d*]pyrimidin-4-yl]oxy}-3-(2-{[1-(2,2,2-trifluoroethyl)-1*H*-pyrazol-5-yl]methoxy}phenyl)propanoic acid,
or addition salts thereof with a pharmaceutically acceptable acid or base,
suitable for simultaneous, sequential or separate use.

2. The combination according to claim 1, wherein the Mcl-1 inhibitor is Compound B: (2*R*)-2-{[(5*Sₐ*)-5-{3-chloro-2-methyl-4-[2-(4-methylpiperazin-1-yl)ethoxy]phenyl}-6-(4-fluorophenyl)thieno[2,3-*d*]pyrimidin-4-yl]oxy}-3-(2-{[2-(2-methoxyphenyl)pyrimidin-4-yl]methoxy}phenyl)propanoic acid, or a pharmaceutically acceptable salt thereof.

3. The combination according to claim 1, wherein the Mcl-1 inhibitor is Compound C: (2*R*)-2-{[(5*Sₐ*)-5-{3-chloro-2-methyl-4-[2-(4-methylpiperazin-1-yl)ethoxy]phenyl}-6-(5-fluorofuran-2-yl)thieno[2,3-*d*]pyrimidin-4-yl]oxy}-3-(2-{[1-(2,2,2-trifluoroethyl)-1*H*-pyrazol-5-yl]methoxy}phenyl)propanoic acid, or a pharmaceutically acceptable salt thereof.

4. The combination according to claim 2, wherein Compound B is to be administered intravenously and Midostaurin is to be administered orally.

5. The combination according to any of claims 1 to 4, further comprising at least one additional anti-cancer agent, for example cytarabine and / or daunorubicin.

6. The combination according to any of claims 1 to 5, in the form of a non-fixed dose combination.

7. The combination according to any of claims 1 to 6, for use in medicine.

8. The combination according to any of claims 1 to 6, for use in the treatment of cancer.

9. The combination according to any of claims 1 to 6, for use according to claim 8, wherein the cancer is acute myeloid leukaemia.

10. The combination according to any of claims 1 to 6, for use according to claim 9, wherein the cancer is acute myeloid leukaemia present in patients carrying a *FLT3* mutation.

11. The combination according to any of claims 1 to 6, for use according to claim 10, wherein the cancer is acute myeloid leukaemia present in patients carrying a *FLT3-ITD* mutation.

12. The combination according to any of claims 1 to 6, for use according to claim 10, wherein the cancer is acute myeloid leukaemia present in patients carrying a *FLT3-TKD* mutation.

13. The combination according to any of claims 1 to 6, for use according to claim 12, wherein said FLT3-TKD mutation comprises FLT3-D835Y or FLT3-F691 mutations within tyrosine kinase domain (TKD) of FLT3.

14. The combination according to any of claims 1 to 6, for use according to any of claims claim 8 to 13, wherein the cancer is resistant to prior therapy.

15. The combination according to any of claims 1 to 6, for use according to claim 14, wherein the cancer is acute myeloid leukaemia resistant to one or more compounds selected from venetoclax, decitabine, daunorubicin, and cytarabine.

16. The combination according to any of claims 1 to 6, for use according to claim 15, wherein the cancer is acute myeloid leukaemia resistant to venetoclax.

17. The combination according to any of claims 1 to 6, for use according to claim 11, wherein said cancer is acute myeloid leukaemia present in patients carrying a *FLT3-ITD* mutation, and said cancer is resistant to venetoclax.

18. The combination according to any of claims 2 or 4 to 6, for use according to any of claims 8 to 17, wherein Midostaurin and Compound B are provided in amounts which are synergistically effective for the treatment of cancer.

19. A pharmaceutical composition comprising the combination according to any of claims 1 to 6, and at least one pharmaceutically acceptable carrier.

20. The use of a combination according to any of claims 1 to 6, in the manufacture of a medicament for the treatment of cancer.

21. The combination according to any of claims 1 to 6, for use according to claim 10, wherein the cancer is acute myeloid leukaemia present in patients carrying a *FLT3-ITD* mutation in the presence of a FLT3-TKD mutation.

## Patentansprüche

1. Kombination aus
(a) Midostaurin oder einem pharmazeutisch verträglichen Salz davon oder einem Komplex davon oder einem Co-Kristall davon oder einem Solvat, einschließlich Hydrat, davon und
(b) einem Mcl-1-Inhibitor, bei welchem es sich um die Verbindung B: (2*R*)-2-{[(5*Sₐ*)-5-{3-Chlor-2-methyl-4-[2-(4-methylpiperazin-1-yl)ethoxy]phenyl}-6-(4-fluorphenyl)thieno[2,3-*d*]pyrimidin-4-yl]oxy}-3-(2-{[2-(2-methoxyphenyl)pyrimidin-4-yl]methoxy}phenyl)propansäure, oder um die Verbindung C: (2*R*)-2-{[(5*Sₐ*)-5-{3-Chlor-2-methyl-4-[2-(4-methylpiperazin-1-yl)ethoxy]phenyl}-6-(5-fluorfuran-2-yl)thieno[2,3-*d*]pyrimidin-4-yl]oxy}-3-(2-{[1-(2,2,2-trifluorethyl)-1*H*-pyrazol-5-yl]methoxy}phenyl)propansäure oder Additionssalze davon mit einer pharmazeutisch verträglichen Säure oder Base handelt,
welche für gleichzeitige, aufeinanderfolgende oder getrennte Verabreichung geeignet ist.

2. Kombination nach Anspruch 1, wobei der Mcl-1-Inhibitor die Verbindung B: (2*R*)-2-{[(5*Sₐ*)-5-{3-Chlor-2-methyl-4-[2-(4-methylpiperazin-1-yl)ethoxy]phenyl}-6-(4-fluorphenyl)thieno[2,3-*d*]pyrimidin-4-yl]oxy}-3-(2-{[2-(2-methoxyphenyl)pyrimidin-4-yl]methoxy}phenyl)propansäure, oder ein pharmazeutisch verträgliches Salz davon ist.

3. Kombination nach Anspruch 1, wobei der Mcl-1-Inhibitor die Verbindung C: (2*R*)-2-{[(5*Sₐ*)-5-{3-Chlor-2-methyl-4-[2-(4-methylpiperazin-1-yl)ethoxy]phenyl}-6-(5-fluorfuran-2-yl)thieno[2,3-*d*]pyrimidin-4-yl]oxy}-3-(2-{[1-(2,2,2-trifluorethyl)-1*H-*pyrazol-5-yl]methoxy}phenyl)propansäure oder ein pharmazeutisch verträgliches Salz davon ist.

4. Kombination nach Anspruch 2, wobei die Verbindung B intravenös verabreicht werden soll und Midostaurin oral verabreicht werden soll.

5. Kombination nach einem der Ansprüche 1 bis 4, weiterhin umfassend mindestens ein weiteres Antikrebsmittel, beispielsweise Cytarabin und/oder Daunorubicin.

6. Kombination nach einem der Ansprüche 1 bis 5 in Form einer nicht-fixierten Dosiskombination.

7. Kombination nach einem der Ansprüche 1 bis 6 zur Verwendung in der Medizin.

8. Kombination nach einem der Ansprüche 1 bis 6 zur Verwendung bei der Behandlung von Krebs.

9. Kombination nach einem der Ansprüche 1 bis 6 zur Verwendung nach Anspruch 8, wobei der Krebs akute myeloische Leukämie ist.

10. Kombination nach einem der Ansprüche 1 bis 6 zur Verwendung nach Anspruch 9, wobei der Krebs akute myeloische Leukämie ist, die bei Patienten auftritt, die eine *FLT3*-Mutation tragen.

11. Kombination nach einem der Ansprüche 1 bis 6 zur Verwendung nach Anspruch 10, wobei der Krebs akute myeloische Leukämie ist, die bei Patienten auftritt, die eine *FLT3-ITD*-Mutation tragen.

12. Kombination nach einem der Ansprüche 1 bis 6 zur Verwendung nach Anspruch 10, wobei der Krebs akute myeloische Leukämie ist, die bei Patienten auftritt, die eine *FLT3-TKD*-Mutation tragen.

13. Kombination nach einem der Ansprüche 1 bis 6 zur Verwendung nach Anspruch 12, wobei die FLT3-TKD-Mutation FLT3-D835Y- oder FLT3-F691-Mutationen innerhalb der Tyrosinkinasedomäne (TKD) von FLT3 umfasst.

14. Kombination nach einem der Ansprüche 1 bis 6 zur Verwendung nach einem der Ansprüche 8 bis 13, wobei der Krebs gegenüber einer vorherigen Therapie resistent ist.

15. Kombination nach einem der Ansprüche 1 bis 6 zur Verwendung nach Anspruch 14, wobei der Krebs akute myeloische Leukämie ist, die gegen eine oder mehrere Verbindungen, ausgewählt aus Venetoclax, Decitabin, Daunorubicin und Cytarabin, resistent ist.

16. Kombination nach einem der Ansprüche 1 bis 6 zur Verwendung nach Anspruch 15, wobei der Krebs akute myeloische Leukämie ist, die gegen Venetoclax resistent ist.

17. Kombination nach einem der Ansprüche 1 bis 6 zur Verwendung nach Anspruch 11, wobei der Krebs akute myeloische Leukämie ist, die bei Patienten auftritt, die eine *FLT3-ITD*-Mutation tragen, und der Krebs resistent gegen Venetoclax ist.

18. Kombination nach einem der Ansprüche 2 oder 4 bis 6 zur Verwendung nach einem der Ansprüche 8 bis 17, wobei Midostaurin und die Verbindung B in Mengen bereitgestellt werden, die für die Behandlung von Krebs synergistisch wirksam sind.

19. Pharmazeutische Zusammensetzung, umfassend die Kombination nach einem der Ansprüche 1 bis 6 und mindestens einen pharmazeutisch verträglichen Träger.

20. Verwendung einer Kombination nach einem der Ansprüche 1 bis 6 bei der Herstellung eines Medikaments zur Behandlung von Krebs.

21. Kombination nach einem der Ansprüche 1 bis 6 zur Verwendung nach Anspruch 10, wobei der Krebs akute myeloische Leukämie ist, die bei Patienten auftritt, die eine *FLT3-ITD*-Mutation in Gegenwart einer FLT3-TKD-Mutation tragen.

## Revendications

1. Combinaison de
(a) Midostaurine, ou un sel pharmaceutiquement acceptable associé, ou un complexe associé, ou un co-cristal associé, ou un solvate, y compris un hydrate, associé, et
(b) un inhibiteur de Mcl-1 qui est le composé B: (2*R*)-2-{[(5*Sₐ*)-5-{3-chloro-2-méthyl-4-[2-(4-méthylpipérazine-1-yl)éthoxy]phényl}-6-(4-fluorophényl)thiéno[2,3-*d*]pyrimidine-4-yl]oxy}-3-(2-{ [2-(2-méthoxyphényl)pyrimidine-4-yl]méthoxy}phényl)acide propanoïque, ou le composé C: (2*R*)-2-{[(5*Sₐ*)-5-{3-chloro-2-méthyl-4-[2-(4-méthylpipérazine-1-yl)éthoxy]phényl}-6-(5-fluorofurane-2-yl)thiéno[2,3-*d*]pyrimidine-4-yl]oxy}-3-(2-{ [1-(2,2,2-trifluoroéthyl)-1*H-*pyrazol-5-yl]méthoxy}phényl)acide propanoïque,
ou des sels d'addition associés avec un acide ou une base pharmaceutiquement acceptable,
convenant à une utilisation simultanée, séquentielle ou séparée.

2. Combinaison selon la revendication 1, dans laquelle l'inhibiteur de Mcl-1 est le composé B: (2*R*)-2-{[(5*Sₐ*)-5-{3-chloro-2-méthyl-4-[2-(4-méthylpipérazine-1-yl)éthoxy]phényl}-6-(4-fluorophényl)thiéno[2,3-*d*]pyrimidine-4-yl]oxy}-3-(2-{[2-(2-méthoxyphényl) pyrimidine-4-yl]méthoxy}phényl)propanoïque, ou un sel pharmaceutiquement acceptable associé.

3. Combinaison selon la revendication 1, dans laquelle l'inhibiteur de Mcl-1 est le composé C: (2*R*)-2-{[(5*Sₐ*)-5-{3-chloro-2-méthyl-4-[2-(4-méthylpipérazine-1-yl)éthoxy]phényl}-6-(5-fluorofurane-2-yl)thiéno[2,3-*d*]pyrimidine-4-yl]oxy}-3-(2-{[1-(2,2,2-trifluoroéthyl)-1*H*-pyrazole-5-yl]méthoxy}phényl)propanoïque, ou un sel pharmaceutiquement acceptable associé.

4. Combinaison selon la revendication 2, dans laquelle le composé B est à administrer par voie intraveineuse et la midostaurine à administrer par voie orale.

5. Combinaison selon l'une quelconque des revendications 1 à 4, comprenant également au moins un agent anti-cancéreux additionnel, par exemple la cytarabine et/ou la daunorubicine.

6. Combinaison selon l'une quelconque des revendications 1 à 5, sous forme de combinaison à dose non fixée.

7. Combinaison selon l'une quelconque des revendications 1 à 6, destinée à être utilisée en médecine.

8. Combinaison selon l'une quelconque des revendications 1 à 6, destinée à être utilisée dans le traitement du cancer.

9. Combinaison selon l'une quelconque des revendications 1 à 6, destinée à être utilisée selon la revendication 8, dans laquelle le cancer est une leucémie myéloïde aiguë.

10. Combinaison selon l'une quelconque des revendications 1 à 6, destinée à être utilisée selon la revendication 9, dans laquelle le cancer est une leucémie myéloïde aiguë présente chez des patients porteurs d'une mutation *FLT3.*

11. Combinaison selon l'une quelconque des revendications 1 à 6, destinée à être utilisée selon la revendication 10, dans laquelle le cancer est une leucémie myéloïde aiguë présente chez des patients porteurs d'une mutation *FLT3-ITD.*

12. Combinaison selon l'une quelconque des revendications 1 à 6, destinée à être utilisée selon la revendication 10, dans laquelle le cancer est une leucémie myéloïde aiguë présente chez des patients porteurs d'une mutation *FLT3-TKD.*

13. Combinaison selon l'une quelconque des revendications 1 à 6, destinée à être utilisée selon la revendication 12, dans laquelle ladite mutation FLT3-TKD comprend des mutations FLT3-D835Y ou FLT3-F691 dans le domaine tyrosine kinase (TKD) de FLT3.

14. Combinaison selon l'une quelconque des revendications 1 à 6, destinée à être utilisée selon l'une quelconque des revendications 8 à 13, dans laquelle le cancer est résistant à une thérapie antérieure.

15. Combinaison selon l'une quelconque des revendications 1 à 6, destinée à être utilisée selon la revendication 14, dans laquelle le cancer est une leucémie myéloïde aiguë résistante à un ou plusieurs composés choisis parmi le vénétoclax, la décitabine, la daunorubicine, la cytarabine.

16. Combinaison selon l'une quelconque des revendications 1 à 6, destinée à être utilisée selon la revendication 15, dans laquelle le cancer est une leucémie myéloïde aiguë résistante au vénétoclax.

17. Combinaison selon l'une quelconque des revendications 1 à 6, destinée à être utilisée selon la revendication 11, dans laquelle ledit cancer est une leucémie myéloïde aiguë présente chez des patients porteurs d'une mutation *FLT3-ITD,* et ledit cancer est résistant au vénétoclax.

18. Combinaison selon l'une quelconque des revendications 2 ou 4 à 6, destinée à être utilisée selon l'une quelconque des revendications 8 à 17, dans laquelle la midostaurine et le composé B sont présents en quantités synergiquement efficaces pour le traitement du cancer.

19. Composition pharmaceutique comprenant la combinaison selon l'une quelconque des revendications 1 à 6, et au moins un véhicule pharmaceutiquement acceptable.

20. Utilisation d'une combinaison selon l'une quelconque des revendications 1 à 6, dans la fabrication de médicaments destinés à être utilisés dans le traitement du cancer.

21. Combinaison selon l'une quelconque des revendications 1 à 6, destinée à être utilisée selon la revendication 10, dans laquelle le cancer est une leucémie myéloïde aiguë présente chez des patients porteurs d'une mutation *FLT3-ITD* en présence d'une mutation FLT3-TKD.
